Europäisches Patentamt

European Patent Office

Office européen des brevets

(19)

(11) Veröffentlichungsnummer : **0 356 699 B1**

(12) **EUROPÄISCHE PATENTSCHRIFT**

(45) Veröffentlichungstag der Patentschrift :
30.09.92 Patentblatt 92/40

(21) Anmeldenummer : 89113625.1

(22) Anmeldetag : 24.07.89

(51) Int. Cl.$^5$ : **A61K 31/44,** A61K 47/10,
A61K 47/12, A61K 47/34,
// (A61K31/44, 31:135)

(54) **Fixe Arzneimittelkombination mit verzögerter Freisetzung.**

(30) Priorität : 30.08.88 DE 3829398

(43) Veröffentlichungstag der Anmeldung :
07.03.90 Patentblatt 90/10

(45) Bekanntmachung des Hinweises auf die
Patenterteilung :
30.09.92 Patentblatt 92/40

(84) Benannte Vertragsstaaten :
AT BE CH DE ES FR GB GR IT LI LU NL SE

(56) Entgegenhaltungen :
EP-A- 0 163 984
EP-A- 0 165 450
WO-A-87/05511
UNLISTED DRUGS, Band 40, Nr. 12, Dezember
1988, Chatham,NJ (US); Seite 321c.

(73) Patentinhaber : **Dr. Rentschler Arzneimittel
GmbH & Co.
Postfach 320 Mittelstrasse 18
W-7958 Laupheim (DE)**

(72) Erfinder : **Nandi, Kumaresh, Dipl.Chem.Dr.
Kurt Schumacher-Str. 15
W-7958 Laupheim (DE)**
Erfinder : **Fischer, Helga
Bachstrasse 17
W-7938 Oberdischingen (DE)**
Erfinder : **Herrmann, Wilfrid, Dr.
Kurt Schumacher-Str. 2
W-7958 Laupheim (DE)**
Erfinder : **Köhne, Hans, Dr. Dipl.-Chem.
Rotbachweg 7
W-7959 Obersulmetingen (DE)**
Erfinder : **Lahr, Wolfgang
Laubacher Strasse 1b
W-1000 Berlin 33 (DE)**
Erfinder : **Schmersahl, Hein Uwe, Dr.
Händelstrasse 36
W-6477 Limeshain 1 (DE)**
Erfinder : **Walch, Hatto, Dr.
Weissdornweg 16
W-7958 Laupheim (DE)**

(74) Vertreter : **Schwabe - Sandmair - Marx
Stuntzstrasse 16
W-8000 München 80 (DE)**

## Beschreibung

Die Erfindung betrifft eine fixe Arzneimittelkombination, enthaltend Nifedipin und Metoprolol, aus der beide Wirkstoffe gemeinsam zeitlich verzögert (retardiert) freigesetzt werden.

Nifedipin ist eine in Wasser praktisch unlösliche Substanz (The Merck Index, 9. Edition, 1976), während Metoprolol als pharmazeutisch bevorzugt verwendetes Tartrat in Wasser sehr leicht löslich ist (Arzneistoffprofile, 2. Ergänzungslieferung, November 1983).

Nifedipin wird in der Pharmazie als Kalziumantagonist, Metoprolol als Beta-1-selektiver Betablocker verwendet; beide Substanzen werden bei der Behandlung cardiovaskulärer Erkrankungen, insbesondere des Bluthochdruckes, nebeneinander verordnet, ohne daß es bisher möglich war, mittels einer fixen Kombination die Therapie einfacher und sicherer zu gestalten.

Der Erfindung liegt die Aufgabe zugrunde, eine fixe Arzneimittelkombination in Form einer festen Darreichungsform zur Verfügung zu stellen, aus der die beiden Wirkstoffe Nifedipin und Metoprolol gemeinsam und zeitlich verzögert (retardiert) freigesetzt werden. Dies ist aus pharmakokinetischer Sicht sinnvoll, da beide Substanzen über eine kurze Plasmahalbwertzeit verfügen. Diese beträgt für Nifedipin 2 bis 4 Stunden und für Metoprolol 3 bis 4 Stunden. Substanzen mit kurzer Plasmahalbwertzeit werden aus dem Organismus entsprechend schnell ausgeschieden, so daß mehrmals täglich die notwendige Arzneistoffdosis verabreicht werden muß, um therapeutisch wirksame Blutspiegel zu erzeugen. Eine Retardierung ist wünschenswert, um die Häufigkeit der Medikamenteneinnahme soweit zu verringern, daß im günstigsten Fall nur einmal pro Tag appliziert werden muß.

Die gemeinsame Retardierung des praktisch wasserunlöslichen Nifedipins gemeinsam mit dem gut wasserlöslichen Metoprolol ist nicht bekannt und da ihre unterschiedlichen physikalischen Eigenschaften gegenläufig sind, erscheinen sie nicht geeignet, zu einer fixen Kombination vereinigt zu werden.

In der DE-OS 34 19 130 wird ein festes Kombinationspräparat mit verbesserter Bioverfügbarkeit beschrieben, enthaltend Nifedipin und einen Betablocker in bestimmten Gewichtsteilen. Dieser Stand der Technik vermittelt die Lehre, daß es als überraschend anzusehen ist, durch gemeinsame Granulation von Nifedipin mit einem der dort genannten Betablocker wie Acebutolol, Atenolol, Nadolol, Propranolol, Pindolol oder Timolol, (vgl. Seite 3, Absatz 4), eine signifikante Erhöhung der Freisetzung und damit eine signifikante Verbesserung der Bioverfügbarkeit für Nifedipin zu erreichen (vgl. Seite 5, Absatz 3). Dieser Sachverhalt wird in Tabelle 1 anhand von Beispiel 1 dargestellt.

Aufgabenstellung und -lösung der DE-OS 34 19 130 sind folglich darin zu sehen, die Lösungsgeschwindigkeit und damit die Freisetzungsgeschwindigkeit des Nifedipins zu erhöhen, also im Vergleich zur vorliegenden Erfindung den gegenteiligen Effekt zu erzielen.

In der DE-OS 36 10 037 wird ein Kombinationspräparat enthaltend Nifedipin mit verzögerter Wirkstofffreisetzung und einen Betablocker, nämlich Mepindolol, beide in bestimmten Gewichtsteilen, beschrieben. Nach der dort vermittelten Lehre werden jeweils getrennt, Mepindolol und Nifedipin mit an sich bekannten Hilfsstoffen granuliert und die gegebenenfalls überzogenen Granulate in Hartgelatine-Kapseln abgefüllt. Mepindolol liegt dabei unretardiert vor. Zur Retardierung des Nifedipins wird die autoretardierende Eigenschaft der an sich unlöslichen Substanz durch Auswahl bestimmter Korngrößen genutzt, ein Effekt, der aus der DE-OS 30 33 919 seit langem bekannt ist.

Die vorliegende Erfindung betrifft dagegen eine fixe Arzneimittelkombination in Form einer festen Darreichungsform, in der das praktisch unlösliche Nifedipin und das gut lösliche Metoprolol bzw. ein Salz davon, zusammen vorliegen, beispielsweise als Tablette, überzogene Tablette, Mehrschichtentablette, Manteltablette oder in gemeinsam granulierter Form als Granulat, wobei Nifedipin in Dosen von 5 bis 240 mg, vorzugsweise 10 bis 120 mg, und Metoprolol in Dosen von 20 bis 400 mg, vorzugsweise 30 bis 250 mg, enthalten sind und beide Wirkstoffe zeitlich verzögert, also retardiert, freigesetzt werden.

Unter Retardierung wird im Sinne der vorliegenden Erfindung die gemeinsame Freisetzung über einen Zeitraum von mindestens 3 Stunden, vorzugsweise mehr als 6 Stunden verstanden. Unter fixer Arzneimittelkombination ist das räumliche, untrennbare Nebeneinanderliegen beider Wirkstoffe zu verstehen, beispielsweise nach gemeinsamer Granulierung oder Mischung zweier getrennt hergestellter Granulate mit anschließender Verpressung oder Herstellung von Preßlingen aus vorgenannten Granulaten zu Zweischichten- oder Mehrschichten- oder Manteltabletten.

Erfindungsgemäß wird die Aufgabe dadurch gelöst, daß Nifedipin in nicht kristallinem Zustand, Metoprolol jedoch in kristallinem Zustand vorliegt, wobei das Freisetzungsverhalten aus der Darreichungsform für beide Substanzen durch die verwendete Matrix bestimmt wird und nicht, wie beispielsweise aus den DE-OSen 36 10 073 und 30 33 919 bekannt, durch die Lösungsgeschwindigkeit der Substanzen.

Es wurde gefunden, daß als Matrizes zur Steuerung der Freisetzung der erfindungsgemäß verwendeten Wirkstoffe ausgewählte, an sich bekannte hydrophile und lipophile Hilfsstoffe in Kombination verwendet werden können, deren alleinige Verwendung nicht oder unvollkommen für eine gemeinsame Retardierung der Wirkstoffe Nifedipin und Metoprolol im Sinne dieser Erfindung geeignet sind. Dazu gehören Acrylharze, beispielsweise die handelsüblichen Eudragit®-Typen mit den Bezeichnungen E, L, S, RL, RS sowie ihre wässrigen Dispersionen, Polyglykole mit mittleren Molekulargewichten zwischen 200 und 35.000, Fettsäuren, Mono-, Di- und Triglyceride solcher Fettsäuren, Fettsäuresalze, Fettalkohole, Polyvinylpyrrolidone als auch Zellulose, Zellulosederivate, Stärke und Stärkederivate.

Zur Herstellung der erfindungsgemäßen fixen Arzneimittelkombination können darüber hinaus Gleit- und Schmiermittel, Zerfallsförderer und Fließregulierungsmittel sowie Überzugsmittel zur Umhüllung der Arzneiformen als Schutz gegen Feuchtigkeit, Licht, Luftsauerstoff oder zur Geschmacksverbesserung Verwendung finden.

Die erfindungsgemäßen Arzneimittelkombinationen enthalten bevorzugt als Matrix oder Hilfsstoffe Stearylalkohol als Fettalkohol, Polyethylenglykol mit mittlerem Molekulargewicht 6000, Polyvinylpyrrolidon (Kollidon® 25), Copolymerisat aus Acrylmethacrylsäureester als Acrylharz (Eudragit ®RS), Stearinsäure als Fettsäure, Magnesiumstearat als Fettsäuresalz, hochdisperses Siliciumdioxyd als Fließregulierungsmittel, Talkum als Gleit- und Schmiermittel sowie Natriumglykolat der Kartoffelstärke (Primojel).

Ein weiterer Vorteil der erfindungsgemäßen Arzneimittelkombinationen besteht darin, daß auf die Verwendung von Füllstoffen, wie sie oft zur Verbesserung der Tablettiereigenschaft festere Arzneiformen Verwendung finden, verzichtet werden kann, so daß die erfindungsgemäßen Formen hinsichtich ihrer Abmessungen ausreichend klein sind und problemlos eingenommen werden können. Ebenso wird auf die Verwendung von Netzmitteln (Tensiden) verzichtet, im Gegensatz zu allen Ausführungsbeispielen des oben genannten Standes der Technik, bei dem Natriumlaurylsulfat und/oder Polysorbat zur Benetzung des stark lipophilen Nifedipins und zu dessen Löslichkeitsverbesserung verwendet werden.

Nachfolgend wird beispielhaft gezeigt, wie die erfindungsgemäßen fixen Kombinationen zusammengesetzt sein können, um zu dem vorteilhaften Effekt der gemeinsamen, zeitlich verzögerten Freisetzung beider Wirkstoffe zu gelangen, im Vergleich zu einer reinen Mischung der beanspruchten Wirkstoffe.

## B e i s p i e l   1

(Vergleich)

| | |
|---|---|
| Metoprololtartrat, crist. | 50,0 g |
| Nifedipin, crist. | 10,0 g |

Beide kristallinen Wirkstoffe wurden miteinander gemischt und die homogene Mischung in Hartgelatinekapseln (Größe 3 zu 60 mg) abgefüllt.

B e i s p i e l   2

| | |
|---|---|
| Nifedipin, crist. | 10,0 g |
| Metoprololtartrat, crist. | 50,0 g |
| Polyethylenglykol | |
| (Mittl. Mol.-Gewicht 6000) | 110,0 g |
| Stearylalkohol | 30,0 g |
| Polyvinylpyrrolidon | 5,0 g |
| (Kollidon ® 25) | |
| Hochdisperse Kieselsäure | 3,0 g |
| (Aerosil ®) | |
| Natriumglykolat der | |
| Kartoffelstärke | 10,0 g |
| (Primojel) | |
| Magnesiumstearat | 2,0 g |

Nifedipin wird in dem bei 70°C geschmolzenen Polyethylenglykol gelöst. Nach Zugabe von Stearylalkohol und Kollidon wird die praktisch klare Schmelze nach dem Erstarren auf eine obere Korngröße von 1,25 mm gebrochen. Das entstandene Granulat wird mit Metoprolol, Aerosil, Primojel und Magnesiumstearat vermischt. Die Mischung wird zu Tabletten mit einem Durchmesser von 9 mm und einem Gewicht von 220 mg verpreßt, in denen Nifedipin zu 10 mg und Metoprolol zu 50 mg pro Tablette vorliegt.

B e i s p i e l   3 a)

| | |
|---|---|
| Nifedipin, crist. | 66,0 g |
| Polyethylenglykol 600 | 762,0 g |
| Stearylalkohol | 198,0 g |
| Polyvinylpyrrolidon | 33,0 g |
| (Kollidon ® 25) | |

## Beispiel 3 b)

| | |
|---|---|
| Metoprololtartrat, crist. | 200,0 g |
| Eudragit® RS | 180,0 g |
| Talkum | 10,0 g |
| Ethanol | 70,0 g |
| Polyvinylpyrrolidon (Kollidon® 25) | 15,0 g |
| Stearinsäure | 30,0 g |

Gemäß Beispiel 2 wird aus Nifedipin, Polyethylenglykol, Stearylalkohol und Kollidon eine Schmelze hergestellt, die nach dem Erstarren zu einem Granulat mit 1,25 mm Kornobergrenze gebrochen wird.

Metoprolol, Eudragit und Talkum werden mit einer Lösung von Polyvinylpyrrolidon und Stearinsäure in Ethanol granuliert. Die feuchte Mischung wird getrocknet und auf 1,25 mm gebrochen.

962,7 g Granulat aus 3a) wird mit dem Granulat aus 3b) unter zusätzlicher Verwendung von Magnesiumstearat und Aerosil ® 972 vermischt. Die Mischung wird zu Tabletten verpreßt. Die fixe Kombination enthält 30 mg Nifedipin und 100 mg Metoprolol pro Tablette.

### Beispiel 4

Granulat gemäß Beispiel 3a) wird mit 11 g Magnesiumstearat und 11 g Aerosil ® R 972 vermischt. Granulat gemäß Beispiel 3b) wird mit 15 g Magnesiumstearat, 10 g Talkum und 8 g Aerosil ® R 972 vermischt.

Über eine 2-stufige Verpressung werden Zweischichttabletten gepreßt. Die fixe Kombination enthält 30 mg Nifedipin und 100 mg Metoprolol pro Tablette.

Die Tabletten nach Beispiel 2 bis 4 können ohne Umhüllung verwendet oder wahlweise mit einem magensaftlöslichen oder magensaftresistenten Überzug versehen werden, unter Verwendung an sich bekannter Hüllstoffe wie Zucker, Acrylharz, Celluloseacetatphthalat, Cellulosederivate, wobei zusätzlich Pigmente und Farbstoffe eingearbeitet werden können.

Das Freisetzungsverhalten der beiden Wirkstoffe aus den erfindungsgemäßen Formen wurde im Vergleich zur reinen Mischung geprüft (Tabelle 1). Es zeigt sich, daß überraschend beide Wirkstoffe über einen Zeitraum von mindestens 3 Stunden bis mehr als 6 Stunden retardiert freigesetzt werden.

EP 0 356 699 B1

## Tabelle 1

Freisetzung in Prozent (kumuliert)

| Zeit (h) | | 1 h | 3 h | 5 h | 7 h |
|---|---|---|---|---|---|
| Beispiel 1 (Vergleich) | Nifedipin | 46,7 | | | |
| | Metoprolol | 103,0 | | | |
| Beispiel 2 | Nifedipin | 36,9 | 81,7 | 97,4 | |
| | Metoprolol | 77,7 | 105,0 | 105,0 | |
| Beispiel 3 | Nifedipin | 45,0 | 86,0 | 101,0 | |
| | Metoprolol | 21,0 | 61,7 | 78,3 | 86,4 |
| Beispiel 4 | Nifedipin | 34,6 | 71,4 | 87,2 | 95,7 |
| | Metoprolol | 38,6 | 68,7 | 85,5 | 99,0 |

**Patentansprüche**

1. Fixe Arzneimittelkombination, enthaltend Nifedipin und Metoprolol oder eines ihrer pharmazeutisch verträglichen Salze, **dadurch gekennzeichnet,** daß Nifedipin in nichtkristalliner Form und Metoprolol in kristalliner Form vorliegen und beide Wirkstoffe gemeinsam über einen Zeitraum von mindestens 3 Stunden bis mehr als 6 Stunden verzögert freigesetzt werden.

2. Arzneimittelkombination nach Anspruch 1, **dadurch gekennzeichnet,** daß Nifedipin in einer Menge von 5 bis 240 mg, vorzugsweise 10 bis 120 mg enthalten ist.

3. Arzneimittelkombination nach Anspruch 1 bis 2, **dadurch gekennzeichnet,** daß Metoprolol in einer Menge von 20 bis 400 mg, vorzugsweise 30 bis 250 mg enthalten ist.

4. Arzneimittelkombination nach Anspruch 1 bis 3, **dadurch gekennzeichnet,** daß als Matrix Stearylalkohol allein und/oder Acrylharze enthalten sind.

5. Arzneimittelkombination nach Anspruch 1 bis 4 in Form von Tabletten oder Manteltabletten oder Mehrschichttabletten oder deren überzogene Formen.

6. Arzneimittelkombination nach Anspruch 1 bis 5 zur Verwendung bei der Behandlung cardiovaskulärer Krankheiten, insbesondere des Bluthochdrucks.

**Claims**

1. A fixed combination of medicaments, containing Nifedipin and Metoprolol or one of their pharmaceutically compatible salts, characterised in that nifedipine is present in non-crystalline and metoprolol in crystalline form and in that both active principles are jointly delay-released over a period of at least three hours up to more than six hours.

2. A combination of medicaments according to claim 1, characterised in that nifedipine is contained in a quantity of 5 to 240 mg and preferably 10 to 120 mg.

6

3. A combination of medicaments according to claim 1 to 2, characterised in that metoprolol is contained in a quantity from 20 to 400 mg and preferably 30 to 250 mg.

4. A combination of medicaments according to claim 1 to 3, characterised in that stearyl alcohol alone and/or acrylic resins are contained as a matrix.

5. A combination of medicaments according to claim 1 to 4, in the form of tablets or encased tablets or multilayer tablets or their coated forms.

6. A combination of medicaments according to claim 1 to 5, for use in the treatment of cardiovascular diseases, particularly high blood pressure.


## Revendications

1. Combinaison fixe de médicaments, contenant de la nifédipine et du métoprolol ou un de leurs sels pharmaceutiquement acceptables, **caractérisée en ce que** la nifédipine se présente sous forme non cristalline et le métoprolol se présente sous forme cristalline, les deux substances actives étant libérées conjointement de manière retardée sur un laps de temps d'au moins 3 heures à plus de 6 heures.

2. Combinaison de médicaments selon la revendication 1, **caractérisée en ce que** la nifédipine est contenue en une quantité de 5 à 240 mg, de préférence de 10 à 120 mg.

3. Combinaison de médicaments selon la revendication 1 ou 2, **caractérisée en ce que** le métoprolol est contenu en une quantité de 20 à 400 mg, de préférence de 30 à 250 mg.

4. Combinaison de médicaments selon les revendications 1 à 3, **caractérisée en ce qu'**elle contient, à titre de matrice, de l'alcool stéarylique seul et/ou des résines acryliques.

5. Combinaison de médicaments selon les revendications 1 à 4, sous forme de comprimés ou de comprimés enrobés ou encore de comprimés multicouches ou bien des formes revêtues de ces comprimés.

6. Combinaison de médicaments selon les revendications 1 à 5, pour l'utilisation dans le traitement des maladies cardio-vasculaires, en particulier de la pression sanguine élevée.